# EUROPÄISCHE PATENTSCHRIFT

(11) **EP 0 695 745 B1**
(45) Veröffentlichungstag und Bekanntmachung des Hinweises auf die Patenterteilung: **08.07.1998**
(21) Anmeldenummer: 95112162.3
(22) Anmeldetag: 02.08.1995
(51) Int. Cl.: C07D 295/02

(54) **Verfahren zur destillativen Gewinnung von N-Ethylpiperazin**
Process for the recovery of N-ethylpiperazine by distillation
Procédé pour la récupération de N-éthylpipérazine par distillation

(30) Priorität: 03.08.1994 DE 4427511
(43) Veröffentlichungstag der Anmeldung: 07.02.1996
(73) Patentinhaber: BASF AKTIENGESELLSCHAFT, 67056 Ludwigshafen (DE)
(72) Erfinder: Malsch, Klaus-Dieter, Dr., D-67105 Schifferstadt (DE); Hutton, Douglas, Dr., D-67117 Limburgerhof (DE); Lang, Ernst, Dr., D-67157 Wachenheim (DE)
(74) Vertreter: Isenbruck, Günter, Dr.

(56) Entgegenhaltungen:
- EP-A- 0 181 536
- EP-A- 0 302 440

## Beschreibung

Die vorliegende Erfindung betrifft ein Verfahren zur destillativen Gewinnung von N-Ethylpiperazin aus Gemischen mit Piperazin, N,N'-Diethylpiperazin, Wasser und untergeordneten Mengen an Ethanol und sonstigen Bestandteilen.

N-Ethylpiperazin ist ein wichtiges Zwischenprodukt für organische Synthesen, das in der Technik nach verschiedenen bekannten Verfahrensvarianten aus Ethanol und Piperazin hergestellt wird. Hierbei erhält man regelmäßig Gemische, die neben dem N-Ethylpiperazin das weniger erwünschte N,N'-Diethylpiperazin, nicht umgesetztes Piperazin, überschüssiges Ethanol, Wasser sowie geringe Mengen einiger Nebenprodukte enthalten.

Wie allgemein bekannt ist und aus den EP-A-181 536 und EP-B1-302 440 hervorgeht, bereitet die Aufarbeitung der vorgenannten Gemische erhebliche Schwierigkeiten, die auf die Anwesenheit von Piperazin und N,N'-Diethylpiperazin zurückzuführen sind. Nach der Lehre EP-B1-302 440 umgeht man die Schwierigkeiten der destillativen Trennung der Gemische, indem man die Destillation unter bestimmten Bedingungen in Gegenwart einer definierten Menge an Wasser in der Destillationsblase vornimmt, wodurch es ermöglicht wird, N,N'-Diethylpiperazin vor dem Piperazin aus dem Ausgangsprodukt diskontinuierlich destillativ zu entfernen.

Allerdings vermag dieses bekannte Verfahren nicht voll zu befriedigen, da bei der Abtrennung des Piperazins sich dieses bei der Kondensation verfestigt und die Kondensationsvorrichtungen blockiert. Zusätzlich wird mit der Zugabe von Wasser in die Destillationsblase auch Ethylpiperazin über Kopf abgezogen und geht damit verloren; außerdem ist auch der erforderliche Energieaufwand zu hoch.

Der Erfindung liegt die Aufgabe zugrunde, bei der destillativen Abtrennung von N-Ethylpiperazin aus den vorbeschriebenen Gemischen die Verfestigung des Piperazins in der Kondensationsvorrichtung zu verhindern und, einen wesentlichen Anstieg der N-Ethylpiperazinkonzentration in der Piperazinfraktion zu vermeiden und gleichzeitig den energetischen Aufwand niedrig zu halten.

Erfindungsgemäß wird dies dadurch erreicht, daß während der Abtrennung der Piperazinfraktion an einem Ort Wasser zugegeben wird, an dem die Piperazinkonzentration größer als die N-Ethylpiperazinkonzentration ist; vorzugsweise wird das Wasser dem Rücklauf auf den Kopf der Kolonne zugegeben. Gemäß einer besonders vorteilhaften Ausbildung des erfindungsgemäßen Verfahrens beträgt die Wassermenge zwischen 10 Gewichts-% und 40 Gewichts-% der aus dem Kondensator ablaufenden Menge.

Das erfindungsgemäße Verfahren beruht auf der überraschenden Beobachtung, daß zum einen Wasser den Schmelzpunkt von Piperazin soweit erniedrigt, daß das Piperazin sich nicht in der Kondensationsvorrichtung verfestigt, und zum anderen, daß die Zugabe von Wasser in den Rücklauf zu keinem wesentlichen Anstieg der N-Ethylpiperazinkonzentration in der Piperazinfraktion führt.

Die Ausgangsgemische fallen bei der in verschiedenen Verfahrensvarianten bekannten Ethylierung von Piperazin mit Ethanol an. Man nimmt diese Reaktionen in Gegenwart von Hydrierkatalysatoren und Wasserstoff, häufig auch in Gegenwart von Wasser und Mineralbasen vor, wobei man das Ethanol zweckmäßigerweise im Überschuß über das Piperazin einsetzt.

Nach Abtrennung des festen Hydrierkatalysators destilliert man das überschüssige Ethanol zusammen mit geringen Mengen Wasser ab. Das so erhaltene Gemisch besteht pro 100 kg aus:
30 - 85 kg N-Ethylpiperazin
7 - 40 kg N,N'-Diethylpiperazin
3 - 20 kg Piperazin
0 - 40 kg Wasser
0 - 5 kg Ethanol
0 - 5 kg Begleitstoffen.

Dies ist das Ausgangsgemisch des erfindungsgemäßen Verfahrens. Ihm wird entsprechend der EP-A-302 440 eine definierte Menge Wasser zugegeben und N,N'-Diethylpiperazin bis auf geringe Mengen abgetrennt. Übrig bleibt ein Gemisch, pro 100 kg bestehend aus:
50 - 90 kg N-Ethylpiperazin
3 - 15 kg N,N'-Diethylpiperazin
4 - 30 kg Piperazin
0 - 10 kg Wasser
0 - 7 kg Begleitstoffen.

Bei der schrittweisen Destillation dieses Gemischs fällt im 1. Destillationsschritt, der bei ca. 98°C beginnt, ein Vorlauf aus Wasser, Piperazin und kleinen Mengen an N-Ethylpiperazin an. Während dieses Destillationsschrittes nimmt die Wasserkonzentration allmählich ab, während die Piperazinkonzentration und in geringem Maße die N-Ethylpiperazinkonzentration ansteigen.

In dem folgenden 2. Destillationsschritt, der sich bei einer Blasentemperatur von etwa 150°C anschließt, wird dem Rücklauf erfindungsgemäß Wasser zugegeben. Man erhält eine Fraktion, die im wesentlichen aus Piperazin, Wasser und zu geringen Teilen aus N-Ethylpiperazin besteht.

In einem 3. Destillationsschritt wird reines N-Ethylpiperazin bei 155°C destillativ abgezogen. Zurück bleibt eine Sumpffraktion, die N,N'-Diethylpiperazin und andere höher siedende Bestandteile enthält.

Die zur Trennung der 4 Fraktionen erforderliche Anzahl an theoretischen Böden liegt zweckmäßigerweise zwischen 15 und 60, vorzugsweise jedoch zwischen 20 und 50. Als Rücklaufverhältnisse empfehlen sich Werte zwischen 0,5 - 2 (1. Schritt), zwischen 5 - 20 (2. Schritt) und zwischen 2 - 5 (3. Schritt).

Dem 3. Destillationsschritt kann ein Zwischenlauf vorgeschaltet werden. Im Zwischenlauf, der bei ca. 155°C beginnt, werden Restmengen an Piperazin und Wasser zusammen mit N-Ethylpiperazin abgezogen. Das im Zwischenlauf enthaltene N-Ethylpiperazin kann z.B. dadurch wiedergewonnen werden, daß der Zwischenlauf zusammen mit der nächsten Charge destillativ aufgearbeitet wird. Der Zwischenlauf wird mit einem Rücklaufverhältnis von 20 - 50 betrieben.

Alle vorstehenden Angaben gelten für das Arbeiten unter Normaldruck (etwa 1 bar), jedoch kann die gesamte Destillation oder es können einzelne Destillationsschritte auch ohne weiteres bei vermindertem oder erhöhtem Druck, etwa im Bereich von 0,05 bis 10 bar ausgeführt werden, da sich das Trennverhalten der Ausgangsgemische sowie der zwischenzeitlich hieraus entstehenden Gemische in diesem Bereich nicht grundsätzlich ändert.

Man kann das erfindungsgemäße Verfahren auch kontinuierlich gestalten, wobei die für das Arbeiten unter Normaldruck erforderliche Anzahl der theoretischen Böden im Verstärkungsteil für den 1. Schritt etwa 10 - 30, für den 2. Schritt etwa 20 - 50 und für den 3. Schritt etwa 5 - 20 beträgt. Für den Abtriebsteil reichen für alle Schritte etwa 10 - 15 theoretische Böden aus.

Apparativ bietet das erfindungsgemäße Verfahren keine Probleme, so daß man es wie üblich in Kolonnen beliebiger Bauart, darunter aus wirtschaftlichen Gründen vorzugsweise in Füllkörperkolonnen oder auch in Kolonnen mit geordneter Packung ausführen kann.

Da mit dem erfindungsgemäßen Verfahren die Aufgabe erfolgreich gelöst wird, Piperazin enthaltende Reaktionsgemische zu fraktionieren und das N-Ethylpiperazin in reiner Form zu gewinnen, ist es nicht mehr erforderlich, das Piperazin beim Syntheseschritt weitgehend abreagieren zu lassen. Hierdurch läßt sich der Anteil des unerwünschten N,N'-Diethylpiperazins auf ein wirtschaftliches Minimum herabsetzen. Zusätzlich kann der energetische und der Zeitaufwand zur Gewinnung von N-Ethylpiperazin wesentlich reduziert werden.

Im Folgenden soll an einem Beispiel das erfindungsgemäße Verfahren detailliert dargestellt werden.

1000 g eines Ausgangsgemisches, bestehend aus:
740 g N-Ethylpiperazin
110 g N,N'-Diethylpiperazin
110 g Piperazin
40 g Wasser
werden in einer Packungskolonne mit etwa 20 theoretischen Trennstufen bei 1 bar fraktioniert destilliert. Dies geschieht in folgende Stufen:

### 1. Stufe

Zwischen 98 und 150°C werden 45 g einer Fraktion mit < 0,1 Gew.-% N,N'-Diethylpiperazin, 3 Gew.-% N-Ethylpiperazin, 78 Gew.-% Wasser und 16 Gew.-% Piperazin abgezogen. Das Rücklaufverhältnis beträgt 1.

### 2. Stufe

Zwischen 150 und 155°C werden 230 g einer Fraktion mit < 0,1 Gew.-% N,N'-Diethylpiperazin, 9 Gew.-% N-Ethylpiperazin, 12 Gew.-% Wasser und 78 Gew.-% Piperazin abgezogen. Dem Rücklauf werden dabei über den Destillationsschritt gleichmäßig verteilt 20 g Wasser zugegeben. Das Rücklaufverhältnis beträgt 10.

### 3. Stufe

Zwischen 155 und 160°C werden 80 g einer Fraktion mit < 0,1 Gew.-% N,N'-Diethylpiperazin, < 0,1 Gew.-% Wasser, 3 Gew.-% Piperazin und 97 Gew.-% N-Ethylpiperazin abgezogen. Das Rücklaufverhältnis beträgt 30.

### 4. Stufe

Bei 160°C werden 490 g einer Fraktion mit > 99 Gew.-% N-Ethylpiperazin abgezogen. Das Rücklaufverhältnis beträgt 3.

Durch diese Vorgehensweise können 66% des eingesetzten N-Ethylpiperazins als Reinprodukt gewonnen werden. Weitere 10% können zurückgeführt und mit der nächsten Charge aufgearbeitet werden.

## Patentansprüche

1. Verfahren zur destillativen Gewinnung von N-Ethylpiperazin aus Gemischen bestehend im wesentlichen pro 100 kg aus 30 - 85 kg N-Ethylpiperazin, 7 - 40 kg N,N'-Diethylpiperazin, 3 - 20 kg Piperazin, 0 - 40 kg Wasser, 0 - 5 kg Ethanol, 0 - 5 kg Begleitstoffen,
**dadurch gekennzeichnet**,
daß während der Abtrennung der Piperazinfraktion Wasser an einem Ort zugegeben wird, an dem die Piperazinkonzentration größer als die N-Ethylpiperazirkonzenkation ist.

2. Verfahren nach Anspruch 1,
**dadurch gekennzeichnet**,
daß das Wasser dem Rücklauf auf den Kopf der Kolonne zugegeben wird.

3. Verfahren nach Anspruch 1 oder 2,
**dadurch gekennzeichnet**,
daß die Wassermenge zwischen 10 Gewichts-% und 40 Gewichts-% der aus dem Kondensator ablaufenden Menge beträgt.

4. Verfahren nach Anspruch 1, 2 oder 3,
**dadurch gekennzeichnet**,
daß die Abtrennung der Piperazinfraktion bei einer Temperatur in der Destillationsblase von etwa 145°C bis 155°C bei einem Druck von etwa 1 bar in der Destillationsblase erfolgt.

5. Verfahren nach einem der vorhergehenden Ansprüche,
**dadurch gekennzeichnet**,
daß nach der Abtrennung der Hauptmenge des Piperazins und vor der Abtrennung des N-Ethylpiperazins ein Zwischenlauf vorgesehen ist, bei dem Restmengen von Piperazin und Wasser zusammen mit N-Ethylpiperazin abgezogen werden und das bei diesem Zwischenlauf gewonnene Gemisch mit der nächsten Charge destillativ aufgearbeitet wird.

## Claims

1. A process for the recovery of N-ethylpiperazine by distillation from mixtures essentially comprising, per 100 kg, 30-85 kg of N-ethylpiperazine, 7-40 kg of N,N'-diethylpiperazine, 3-20 kg of piperazine, 0-40 kg of water, 0-5 kg of ethanol and 0-5 kg of accompanying substances, wherein, while the piperazine fraction is being separated off, water is added at a location at which the piperazine concentration is greater than the N-ethylpiperazine concentration.

2. A process as claimed in claim 1, wherein the water is added to the reflux to the top of the column.

3. A process as claimed in claim 1 or 2, wherein the amount of water is from 10 to 40% by weight, based on the amount flowing out of the condenser.

4. A process as claimed in claim 1, 2 or 3, wherein the piperazine fraction is separated off at a temperature in the still of from about 145 to 155°C at about 1 bar in the still.

5. A process as claimed in any of the preceding claims, wherein, after the main amount of the piperazine has been separated off and before the N-ethylpiperazine has been separated off, an intermediate cut is provided in which residual amounts of piperazine and water are removed together with N-ethylpiperazine and the mixture obtained in this intermediate cut is worked up with the next batch by distillation.

## Revendications

1. Procédé pour l'obtention par distillation de N-éthylpipérazine à partir de mélanges consistant essentiellement pour 100 kg en 30 - 85 kg de N-éthylpipérazine, 7 - 40 kg de N,N'-diéthylpipérazine, 3 - 20 kg de pipérazine, 0 - 40 kg d'eau, 0 - 5 kg d'éthanol, 0 - 5 kg d'impuretés,
caractérisé par le fait que, pendant la séparation de la fraction pipérazine, on ajoute de l'eau en un point où la concentration de pipérazine est supérieure à la concentration de N-éthylpipérazine.

2. Procédé selon la revendication 1, caractérisé par le fait que l'eau est ajoutée dans le retour de liquide en tête de colonne.

3. Procédé selon la revendication 1 ou 2, caractérisé par le fait que la quantité d'eau s'élève entre 10 % en poids et 40 % en poids de la quantité s'écoulant du condenseur.

4. Procédé selon la revendication 1, 2 ou 3, caractérisé par le fait que la séparation de la fraction pipérazine s'effectue à une température dans l'ampoule à distiller d'environ 145°C à 155°C sous une pression d'environ 1 bar dans l'ampoule à distiller.

5. Procédé selon l'une quelconque des revendications précédentes, caractérisé par le fait que, après la séparation de la majeure partie de la pipérazine et avant la séparation de la N-éthylpipérazine, il est prévu une phase intermédiaire dans laquelle les quantités résiduaires de pipérazine et d'eau sont éliminées conjointement avec la N-éthylpipérazine et le mélange obtenu dans cette phase intermédiaire est retraité par distillation avec la charge suivante.
